Europäisches Patentamt

European Patent Office

Office européen des brevets

(19)

(11) Veröffentlichungsnummer: **0 033 860**
**B1**

(12) # EUROPÄISCHE PATENTSCHRIFT

(45) Veröffentlichungstag der Patentschrift: **18.09.85**

(21) Anmeldenummer: **81100347.4**

(22) Anmeldetag: **17.01.81**

(51) Int. Cl.⁴: **C 07 B 31/00, C 07 C 29/17,
C 07 C 29/19, C 07 C 29/132,
C 07 C 5/02, C 07 D 211/02,
C 07 D 307/08, C 07 C 1/253,
C 07 C 85/12, C 07 C 87/28,
C 07 C 85/11**

(54) **Verfahren zur katalytischen Hydrierung organischer Verbindungen.**

(30) Priorität: **30.01.80 DE 3003254**

(43) Veröffentlichungstag der Anmeldung:
**19.08.81 Patentblatt 81/33**

(45) Bekanntmachung des Hinweises auf die
Patenterteilung:
**18.09.85 Patentblatt 85/38**

(84) Benannte Vertragsstaaten:
**BE CH DE FR GB IT LI NL**

(56) Entgegenhaltungen:
**DE-A-2 240 849
DE-B-1 270 007
FR-A-2 343 720
US-A-3 290 348
US-A-3 819 734**

(73) Patentinhaber: **HOECHST
AKTIENGESELLSCHAFT
Postfach 80 03 20
D-6230 Frankfurt am Main 80 (DE)**

(72) Erfinder: **Kümmerle, Kurt, Dr.
Unter den Eichen 1
D-6233 Kelkheim (Taunus) (DE)**
Erfinder: **Heise, Hartmut, Dr.
Am Rehsteig 8
D-6232 Bad Soden am Taunus (DE)**

EP 0 033 860 B1

Courier Press, Leamington Spa, England.

## Beschreibung

In der deutschen Patentschrift 2 240 849 wird ein Verfahren zur Herstellung von Aminobenzolen, die am aromatischen Kern durch empfindliche Substituenten, wie Halogenatome, aliphatische Sulfonyl- oder Halogenalkylgruppen substituiert sein können, durch katalytische Reduktion der entsprechenden Nitrobenzolverbindungen in wäßriger Phase an Nickel-Kontakten unter Zusatz von Promotoren beschrieben. Als Promotoren werden bei diesem Verfahren u.a. vollkommen bzw. weitgehend neutralisierte wäßrige Lösungen der Sauerstoffsäuren des drei- bzw. fünfwertigen Phosphors verwendet.

Bei diesem bekannten Verfahren muß der eingesetzte Promotor die Fähigkeit und Möglichkeit besitzen, als Puffersubstanz wirken zu können. Auch bei Zusatz eines wassermischbaren Lösungsmittels wird dieses nur in einem solchen Umfang zugesetzt, daß die Dissoziationswirkung des vorhandenen Wassers in ausreichendem Maße erhalten bleibt.

In der französischen Patentanmeldung 2 343 720 wird die Herstellung von 3-Hydroxybuttersäurearylamiden durch katalytische Hydrierung von N-Acetoacetylarylamiden in flüssiger Phase mittels eines Hydrierungskatalysators unter Zusatz von Trialkylphosphaten, Aminen oder Phosphinen als Promotoren beschrieben.

Während somit die aus den beiden genannten Literaturstellen bekannten Verfahren auf die Reduktion aromatischer Nitroverbindungen bzw. auf die Hydrierung von N-Acetoacetylarylamiden beschränkt sind, wurde nun überraschenderweise gefunden, daß man organische Verbindungen ohne Beschränkung auf bestimmte Klassen oder Systeme in homogener oder heterogener Phase mittels eines Hydrierungskatalysators unter Zusatz eines Promotors katalytisch hydrieren kann, wobei die Hydrierung von Nitroverbindungen und die Hydrierung von Cyclopentadien zu Cyclopenten ausgeschlossen ist, indem man als Hydrierungskatalysator Nickel, Palladium, Platin, Rhodium, Iridium oder Ruthenium, und als Promotor einen Ester der Sauerstoffsäuren des dreiwertigen Phosphors oder eine neutralisierte wäßrige Lösung der Sauerstoffsäuren des drei- oder fünfwertigen Phosphors verwendet.

Daraus folgt, daß überraschenderweise die Aktivierung von Nickel-Katalysatoren durch Sauerstoffsäuren des drei- oder fünf-wertigen Phosphors nicht auf die mit relativ hoher Wärmetönung ablaufenden katalytischen Reduktionen aromatischer Nitroverbindungen beschränkt ist, sondern vielmehr bei allen Typen von Hydrierungsprozessen bewirkt wird. Ferner ergab sich daraus die Erkenntnis, daß durch die genannten Promotoren eine Verstärkung der Aktivität aller Hydrierungskatalysatoren, das heißt sowohl der Träger-Katalysatoren auf Kontaktbasis Nickel, Palladium, Platin, Rhodium, Iridium oder Ruthenium als auch der gängigen Skelett-Katalysatoren, für alle Hydrierungsreaktionen erreicht wird und daß Ester der Sauerstoffsäuren des drei-wertigen Phosphors bzw. neutralisierte Lösungen der Sauerstoffsäuren des drei- oder fünfwertigen Phosphors besonders wirksame Promotoren für katalytische Hydrogenoseriungen und Hydrogenolysen sind.

Bei dem erfindungsgemäßen Verfahren liegt gegenüber dem aus der zitierten deutschen Patentschrift 22 40 849 bekannten Verfahren ein grundsätzlich anderer Katalysator/Promotor-Mechanismus vor, der in seiner positiven Auswirkung auf die katalytische Hydrierung von so verschiedenen organischen Systemen, auf welche das erfindungsgemäße Hydrierverfahren Anwendung findet, nicht vorhersehbar war.

Aus der aus der genannten französischen Patentanmeldung bekannten Verwendung von u.a. Trialkylphosphaten als Promotoren konnte auf die Eignung von Estern der Sauerstoffsäuren des dreiwertigen Phosphors beim erfindungsgemäßen Verfahren nicht geschlossen werden.

Der beim erfindungsgemäßen Verfahren eingesetzte Promotor kann flüssig oder fest sein. Im letztgenannten Fall wird er vorteilhalterweise in gelöster Form eingesetzt. Die Konzentration des Promotors — bezogen auf die Masse der zu hydrierenden Ausgangsverbindung — kann sich in erheblich breiten Grenzen bewegen. Im allgemeinen liegt die Konzentration zwischen 1 und 10 Gew.-%. Sie kann sich jedoch auch unterhalb von 1% bewegen.

Durch den Promotor-Zusatz wird eine Erniedrigung der Anspringtemperatur bzw. eine Reduzierung des Reaktionsdruckes und/oder eine Verkürzung der Reaktionszeit erreicht. Hydrierungen können somit schonender sowie wirtschaftlicher und erforderlichenfalls selektiv durchgeführt werden.

Die zu hydrierende Substanz kann beispielsweise der Stoffklasse der Alkine, Olefine, Cycloalkene, Aromaten, Carbonylverbindungen, Nitrile, Azokörper, Oxim-Verbindungen oder ungesättigter heterocyclischer Stoffsysteme angehören.

Die erfindungsgemäße Hydrierung wird entweder in homogener Phase (Lösung) oder in heterogener Phase durchgeführt. Die Reaktionsbedingungen hinsichtlich Temperatur und Druck richten sich nach der Stoffklassenzugehörigkeit bzw. der individuellen Konstitution der zu hydrierenden Substanz, wobei in der Regel Hydriertemperaturen unterhalb 150°C und Gesamtdrucke von ca. 50 bar angewendet werden.

Das Verfahren wird durch die nachstehenden Beispiele näher erläutert. Die Konzentrationsangaben beziehen sich auf die Masse der zu hydrierenden Verbindung.

### Beispiel 1

2 Mol 2-Butin-1,4-diol werden in 500 g Methanol in Gegenwart von 1% Nickel-Katalysator bei einem Druck von 45 bar hydriert. Die Reaktion springt bei ca. 80°C an und wird bei 80—85°C weitergeführt. Nach ca. 45 Minuten Hydrierzeit wird n-Butan-1,4-diol in einer Ausbeute von ca. 94% der Theorie erhalten.

Wird dem Ausgangsgemisch 1% Phosphorsäure in Form einer mit wäßriger Natronlauge neutralisierten Lösung zugegeben, so springt die Hydrierung schon bei 70°C an und ist bereits nach ca. 30 Min. beendet, wobei das erhaltene n-Butan-1,4-diol in einer Ausbeute von ca. 94% der Theorie gewonnen wird.

### Beispiel 2

8 Mol Cyclohexen werden in Gegenwart von 1% Nickelkatalysator bei einem Druck von 40 bar hydriert. Die Reaktion springt bei 100°C an und wird bei 120—123°C zu Ende geführt. Nach einer Reaktionszeit von ca. 120 Minuten wird Cyclohexan in einer Ausbeute von über 97% der Theorie erhalten.

Wird der zu hydrierenden Substanz 1% phosphorige Säure in Form einer mit wäßriger Natronlauge neutralisierten Lösung zugesetzt, so liegt die Anspringtemperatur der Hydrierung schon bei 85°C. Die Hydrierzeit bei 120—123°C beträgt nur noch ca. 90 Minuten und die Ausbeute an Cyclohexan beläuft sich auf mehr als 97% der Theorie.

### Beispiel 3

2 Mol Phenol werden in 600 g Methanol in Gegenwart von 1% Nickel-Katalysator bei einem Druck von 50 bar hydriert. Die Reaktion beginnt bei 138°C und wird bei 140—142°C beendet. Nach einer Reaktionszeit von 205 Minuten wird Cyclohexanol in einer Ausbeute von über 95% der Theorie isoliert.

Wird dem Ausgangsgemisch 1% Phosphorige Säure in Form einer mit wäßriger Natronlauge neutralisierten Lösung zugegeben, so springt die Hydrierung schon bei 120°C an und ist bei einer Reaktionstemperatur von 140—142°C bereits nach 160 Minuten beendet. Die Ausbeute an Cyclohexanol beträgt über 95% der Theorie.

### Beispiel 4

10 Mol Furan werden in Gegenwart von 1% Nickelkatalysator bei einem Druck von 40 bar hydriert. Die Reaktion springt bei 88°C an und ist bei einer Temperatur von 95—100°C nach 120 Minuten beendet. Das hierbei gebildete Tetrahydrofuran wird in einer Ausbeute von über 97% der Theorie erhalten.

Wird der Ausgangssubstanz 1% Natriumhydrogenphosphat, das mit wäßriger Natronlauge neutralisiert wird, zugegeben, so beginnt die Wasserstoffaufnahme bereits bei 80°C und die Hydrierung ist bei einer Temperatur von 95—100°C schon nach 80 Minuten abgeschlossen. Die Ausbeute an Tetrahydrofuran beträgt hierbei über 97% der Theorie.

### Beispiel 5

2 Mol D,L-Campher sollten in 500 g Tetrahydrofuran in Gegenwart eines gealterten Nickel-Katalysators (Katalysator-Konzentration: 1%) bei einer Temperatur von 135°C und einem Druck von 50 bar hydriert werden. Selbst nach Erhöhung der Temperatur auf 145°C und Steigerung des Wasserstoffdruckes aud 60 bar kam die Hydrierung nich in Gang.

Wird dem Ausgangsgemisch bei Verwendung desselben gealterten Nickel-Katalysators 1% Natriumdihydrogenphosphat, das mit wäßriger Natronlauge neutralisiert ist, als Promotor zugesetzt, so springt die Reaktion bei 125°C an. Die Hydrierung wird bei 135°C weitergeführt und ist nach einer Dauer von ca. 30 Minuten beendet. Die Ausbeute an hydriertem Endprodukt (Camphan) beträgt 94% der Theorie.

### Beispiel 6

2 Mol Benzonitrol werden in 700 g p-Xylol in Gegenwart von 2,5% Palladium-Katalysator bei einem Druck von 45 bar hydriert. Die Wasserstoffaufnahme beginnt bei 80°C. Die Hydrierung wird bei 105°C weitergeführt und ist nach einer Reaktionszeit von 55 Minuten beendet. Die Ausbeute an Benzylamin ist größer als 94% der Theorie.

Wird dem Ausgangsgemisch 2,5% Natriumdihydrogenphosphat, das mit wäßriger Nationlauge neutralisiert wird, zugesetzt, so springt die Hydrierung schon bei 68°C an. Die Reaktion wird bei 105°C weitergeführt und ist nach einer Reaktionszeit von ca. 40 Minuten bereits beendet. Benzylamin wird in einer Ausbeute von über 94% der Theorie isoliert.

In der US—PS 3 819 734 wird ein neuer Typ eines Katalysators und ein Verfahren zur selektiven Hydrierung von Cyclopentadien zu Cyclopenten unter Verwendung dieser neuen Katalysatorart beschrieben. Dieserneue Katalysatortyp besteht aus Nickel/Magnesiumoxalat oder Nickel/Zinkoxalat und jeweils einem Liganden aus der Gruppe der Phosphine und Phosphite. Bei dem bekannten System stellen Metall/Träger/Ligand ein makroskopisch einheitliches Stoffsystem dar, in welches die einzelnen Komponenten voll integriert sind, während erfindungsgemäß eine Mischung aus selbständigen Komponenten (Katalysator und Promotor) mit loser, d.h. nicht gebundener Wechselwirkung zur Anwendung gelangt. Der aus der zitierten US—PS bekannte Katalysator unterscheidet sich somit von dem erfindungsgemäß angewandten Katalysator/Promotor-System sowohl in der Morphologie als auch in der Struktur und auch in der Wirkung insofern, als das Katalysator-System der US—PS selektiv wirkt (Reduktion des Cyclopentadiens zum Cyclopenten), während das erfindungsgemäß eingesetzte Katalysator/Promotor-System zur Hydrierung der verschiedenartigsten organischen Verbindungstypen geeignet ist.

**Patentansprüche**

1. Verfahren zur katalytischen Hydrierung von organischen Verbindungen, wobei die Hydrierung von Nitroverbindungen und die Hydrierung von Cyclopentadien zu Cyclopenten ausgeschlossen ist, in homogener oder heterogener Phase mittels eines Hydrierungskatalysators unter Zusatz eines Promotors, dadurch gekennzeichnet, daß der Hydrierungskatalysator Nickel, Palladium, Platin, Rhodium, Iridium oder Ruthenium, und der Promotor ein Ester der Sauerstoffsäuren des drei-wertigen Phosphors oder eine neutralisierte wäßrige Lösung der Sauerstoffsäuren des drei- oder fünfwertigen Phosphors ist.

2. Verfahren nach Anspruch 1, dadurch gekennzeichnet, daß der Promotor in flüssiger oder gelöster Form eingesetzt wird.

3. Verfahren nach Anspruch 1 oder 2, dadurch gekennzeichnet, daß man die Hydrierung in Gegenwart von 1 bis 10 Gew.-% des Promotors, bezogen auf die zu hydrierende Ausgangsverbindung, durchführt.

**Revendications**

1. Procédé d'hydrogénation catalytique de composés organiques, l'hydrogénation de composés nitrés et l'hydrogénation du cyclopentadiène en cyclopentène étant exclue, en phase homogène ou hétérogène, à l'aide d'un catalyseur d'hydrogénation sous addition d'un promoteur, caractérisé en ce que le catalyseur d'hydrogénation est le nickel, le palladium, le platine, le rhodium, l'iridium ou le ruthénium, et que le promoteur est un ester des acides oxygénés du phosphore trivalent ou une solution aqueuse neutralisée des acides oxygénés du phosphore trivalent ou pentavalent.

2. Procédé selon la revendication 1, caractérisé en ce que le promoteur est utilisé sous forme liquide ou dissoute.

3. Procédé selon l'une des revendications 1 ou 2, caractérisé en ce que l'hydrogénation est réalisée en présence de 1 à 10% en poids du promoteur, sur la base du composé de départ à hydrogéner.

**Claims**

1. A process for the catalytic hydrogenation of organic compounds, the hydrogenation of nitro compounds and the hydrogenation of cyclopentadiene to cyclopentene being excluded, in homogeneous or heterogeneous phase with a hydrogenation catalyst in the presence of a promotor, wherein the hydrogenation catalyst is nickel, palladium, platinum, rhodium, iridium or ruthenium, and the promotor is an ester of the oxyacids of the tri-valent phosphorus or a neutralized aqueous solution of the oxyacids of the tris- or penta-valent phosphorus.

2. The process as claimed in claim 1, wherein the promotor is used in liquid or dissolved form.

3. The process as claimed in claim 1 or 2, wherein the hydrogenation is performed in the presence of 1 to 10 percent by weight of the promotor, referred to the starting compound to be hydrogenated.